# EUROPEAN PATENT SPECIFICATION

(11) **EP 2 016 038 B1**
(45) Date of publication and mention of the grant of the patent: **11.11.2009**
(21) Application number: 06745255.7
(22) Date of filing: 03.04.2006
(51) Int. Cl.: C07C 51/09, C07C 63/26, C08J 11/04, C08L 67/02

(54) **RECOVERY OF AROMATIC DICARBOXYLIC ACIDS FROM WASTE POLYESTER RESIN IN THE PRESENCE OF A POLYAMIDE**
RÜCKGEWINNUNG VON AROMATISCHEN DICARBONSÄUREN AUS POLYESTERHARZABFÄLLEN IN GEGENWART EINES POLYAMIDS
RECUPERATION D'ACIDES DICARBOXYLIQUES AROMATIQUES A PARTIR DE DECHETS DE RESINE DE POLYESTER EN PRESENCE D'UN POLYAMIDE

(43) Date of publication of application: 21.01.2009
(73) Proprietor: Cobarr S.r.l., 15057 Tortona (Alessandria) (IT)
(72) Inventor: AL GHATTA, Hussain, I-03014 Fiuggi (IT); HRONEC, Milan, 821 05 Brastislava (SK)
(74) Representative: Gerbino, Angelo
(86) International application number: PCT/IT2006/000220
(87) International publication number: WO 2007/113872

(56) References cited:
- GB-A- 839 371
- US-A- 4 605 762
- US-A- 5 413 681
- US-A1- 2004 068 055

## Description

### Background

Producers of polyethylene terephthalate and its copolymers, collectively known as PET, have already developed processes for recycling PET scrap from polyester film, fiber and bottle production. The methods for recovering terephthalic acid and ethylene glycol from PET are based on depolymerization, for example by hydrolysis under conditions of neutral pH or in the presence of acids or bases, by acetolysis, methanolysis or glycolysis. U.S. Patent No. 6,670,503 describes a method of recovering terephthalic acid from PET in the absence of water, with a reagent consisting of one or more metal salts of an acid weaker than terephthalic acid, until a watersoluble compound is obtained, then subsequently carrying out dissolution in water and acidification.

According to United States Patent No. 6,239,310, PET is heated in an aqueous solution at temperatures from 150 °C to 280 °C with a reagent substance chosen from the group comprising bicarbonates of ammonia and alkali metals, ammonium carbamate and urea. In United States Patent No. 6,545,061, a process is disclosed for depolymerizing and purifying recyclable PET comprising acetolysis to form terephthalic acid and ethylene glycol diacetate.

United States Patent No. 6,562,877 claims a process for depolymerizing recycled colored and contaminated aromatic PET articles with acceptable color characteristics, by depolymerizing said recycled articles in acetic acid at high temperatures (160 - 250 °C).

United States Patent No. 6,723,873 discloses a process for recovering terephthalic acid from PET by ammoniolysis. In this process, PET is reacted with ammonium hydroxide to form diammonium terephthalate which is then converted to terephthalic acid by heating at a temperature from about 225 °C to about 300 °C. Depolymerization of PET by hydrolysis at a high temperature and pressure in the absence of a base or acid, is known, see for example United States Patent No. 4,578,502, or United States Patent No. 4,605,762.

Although various processes are available for hydrolyzing PET waste, the purification of recovered terephthalic acid typically requires several process steps to remove dyes, pigments and other impurities including inorganic compounds and salts. In addition, recrystallization and hydrogenation over noble metal catalysts are usually used to purify terephthalic acid.

Starting materials for making terephthalic acid include such things as mixtures of polyester film, fiber and PET bottle waste. This raw material can also contain metals, labels, lighter plastics, glass, rock and other heavy impurities. These contaminants are removed by such pretreatment processes as skimming or decantation. Depending on the types of contaminants present, bulk solids can also be separated after the hydrolysis process. In a hydrolysis process, PET scrap ground into small particles is depolymerized at temperatures ranging from about 230 °C to about 300 °C and usually at pressures sufficient to maintain a liquid phase.

Recycled PET waste used as feed to the hydrolysis process often contains traces of corrosion product, e.g. PVC (50 - 200 ppm), metal chlorides, or halogen containing compounds which at the temperature of PET hydrolysis decompose to very corrosive chlorine and/or halogen compounds which attack the reactor. Since the PVC contaminants of the recycled articles can not be absolutely excluded, the hydrolytic processes must use expensive titanium based equipment to avoid corrosion and high metal content and discoloring of the produced terephthalic acid.

### Summary of the invention

This specification discloses a method of recovering aromatic dicarboxylic acids from aromatic polyester resins containing less than 2 500 ppm of halogen containing compounds, comprising the step of depolymerizing the aromatic polyester resin in the presence of water at temperatures in the range of 230 °C to 300 °C wherein the reaction occurs in the presence of 1.0 to 10 wt % of a polyamide wherein the polyamide is selected from the group consisting of the repeating unit of amino caproic acid and the reaction product of A and D, wherein A is a residue of a dicarboxylic acid comprising adipic acid, isophthalic acid, terephthalic acid, 1,4-cyclohexanedicarboxylic acid, or naphthalene dicarboxylic acid, and D is a residue of a diamine comprising m-xylylene diamine, p-xylylene diamine, hexamethylene diamine, ethylene diamine, or 1,4 cyclohexanedimethylamine. It is further disclosed that the depolymerization reaction proceed in the presence of 1 to 8 wt % of activated charcoal calculated to the amount of polyester resin. It is also disclosed that the depolymerized material may be subsequently passed through a fixed-bed adsorber filled with activated charcoal at a temperature in the range of 50 °C below the depolymerization temperature to the depolymerization temperature. It is further disclosed that the ratio of the amount of water at the end of depolymerization to the amount of aromatic polyester resin present in the reaction mixture is between 4 : 1 to 10 : 1 and that the amount the water at the beginning of the reaction may optionally contain ethylene glycol at an amount less than or equal to 10 weight percent with respect to the water plus ethylene glycol mixture. It is further disclosed that the aromatic dicarboxylic acid can be separated from the reaction mixture cooled to the temperature in the range from 180 to 90 °C.

### Detailed description

It has been found that it is possible to produce terephthalic acid with metal contents below 10 ppm using a) the neutral hydrolytic depolymerization of PET waste containing less than 2,500 ppm of halogen containing compounds, b) a stainless steel reactor, c) the presence of 1.0 to 10 wt % of polyamide calculated to PET(in particular aliphatic based polyamides such as PA 6, PA 66, and polyamides based on MXDA as amine components, most notably MXD6 and PET/MXD6 blends or polyester polyamides). The hydrolytic depolymerization of PET waste proceeds usually in one step at temperatures between 230 °C and 300 °C, preferably at 260 - 275 °C and at a pressure sufficient to maintain a liquid phase.

To avoid the purification of recovered terephthalic acid, the hydrolytic depolymerization of PET is performed in the presence of activated charcoal (1 - 7 wt % to PET) or the reaction mixture is passed through a fixed-bed adsorber filled with granulated activated charcoal after the depolymerization reaction.

The disclosed process is for recovering terephthalic acid from waste polyester film, fiber, bottles, manufacturing residues and other manufactured articles, which contain less than 2,500 ppm of corrosion compounds, e.g. PVC, metal chlorides, halogenated compounds.

In the preferred embodiment, material waste such as PET is broken into chips or fragments that may be produced from mixtures of articles of different colors and different origin including articles made of or containing polyester resin mixed with polyamides such as poly (m-xylylene adipamide) or other polymers. Of particular interest are those articles in the form of multi-layer films or multi-layer bottles in which at least one layer is made of co(polyalkylene terephthalate) and one layer is made of poly(m-xylylene adipamide), or mixtures thereof with polyalkylene terephthalate.

The polyamides suitable for this invention can be described as comprising the repeating unit of amino caproic acid or A-D, wherein A is the residue of a dicarboxylic acid comprising adipic acid, isophthalic acid, terephthalic acid, 1,4-cyclohexanedicarboxylic acid, or naphthalene dicarboxylic acid, and D is a residue of a diamine comprising m-xylylene diamine, p-xylylene diamine, hexamethylene diamine, ethylene diamine, or 1,4 cyclohexanedimethylamine.

These polyamides can also be described as the comprising at least one reaction product selected from the group consisting of the reaction product of amino caproic acid with itself and/or the reaction product of a residue of a dicarboxylic acid comprising adipic acid, isophthalic acid, terephthalic acid, 1,4-cyclohexanedicarboxylic acid, or naphthalene dicarboxylic acid with a residue of a diamine comprising m-xylylene diamine, p-xylylene diamine, hexamethylene diamine, ethylene diamine, or 1,4 cyclohexanedimethylamine.

Those skilled in the art will recognize many of the combinations as well known commercially available polyamides. The reaction product of the residue of sebacic acid with hexamethylene diamine is nylon 6.10 and the reaction product of the residue of adipic acid and hexamethylene diamine is nylon 6.6. Nylon 6.12 is another nylon which benefits from the invention. Nylon 6 is a special type of polyamide having the formula of H₂N-(CH₂)₅-COOH and is made by the opening of caprolactam ring and then reacting' or polymerizing the resulting amino caproic acid with itself. Although nylon 6 is a preferred polyamide polymer, the most preferred polyamide polymer is the reaction product of the residues of adipic acid and m-xylylene diamine, known as poly-m-xylylene adipamide. This product is commercially known as MXD6 or nylon MXD6.

The reaction is preferably carried out in a stainless steel reactor for 30 - 120 minutes and can be performed continuously or discontinuously. The hydrolytic depolymerization may be conducted in water, mixtures of ethylene glycol and water (up to 10 wt %), or steam which is purged into the reaction system. The weight ratio of PET/water is from 1 : 4 to 1 : 10, preferably 1 : 5 to 1 : 7. The depolymerization process can be carried out in air or more preferably inert gas, which is most preferred. The produced terephthalic acid is separated from the liquid phase by conventional methods, for example after cooling by filtration.

Terephthalic acid prepared according to the present invention contain no polyamides or amino compounds in detectable amounts. The iron content is below 10 ppm. The L* color parameters of recovered terephthalic acid samples prepared, even those prepared from the mixture of green, blue and brown colored PET waste, are above 82 - 84.

The following examples illustrate but do not limit the scope of the present invention.

### Example 1

270 g of colored PET bottle scrap, 3.25 wt % of poly(m-xylylene adipamide), 350 ppm of iron compounds and inorganic salts, and 1,700 g deionized water were weighed into a 3 1 stainless steel rocking autoclave. Inside the reactor, a basket was filled with 20 g of granulated (0.6 - 0.7 mm) activated charcoal. The reactor was twice purged with nitrogen and heated at 270 °C for 90 minutes. After cooling to approximately 90 °C, the resultant terephthalic acid was separated by filtration and then filter washed (3 x 300 ml) with warm (approximately 75 °C) deionized water. The product was dried in an oven overnight at 95 °C. Terephthalic acid was obtained in 89.8 % mol yield and contained 4.6 ppm of iron.

### Comparative experiment 1

The procedure of Example 1 was repeated, except that 270 g of recycled clear PET without the polyamide was used. The resultant terephthalic acid contained 31.7 ppm of iron.

### Comparative experiment 2

27 g of other sources PET scrap and 150 g deionized water was weighed into a 250 ml stainless steel rocking autoclave. Inside the reactor a basket was filled with 0.5 g of granulated (0.6 - 0.7 mm) activated charcoal. The reactor was twice purged with nitrogen and heated at 270 °C for 60 minutes. Terephthalic acid isolated by filtration, washed with ca. 75 °C deionized water ( 3 x 50 ml) and dried at 95 °C overnight contained 35.3 ppm of iron.

### Example 2

The procedure of comparative experiment 2 was repeated except that 0.70 g PVC (2590 ppm) and 1.35 g polyamide MXD6 was added. The recycled terephthalic acid contained 9.5 ppm of iron.

### Example 3

The procedure of example 2 was repeated except that no PVC was added. The isolated terephthalic acid contained 8.34 ppm of iron.

The results of Experiments 2 and 3 clearly demonstrate the positive effects of polyamide on the content of iron in the recovered terephthalic acid, even in the presence of huge concentrations of PVC in the depolymerization process.

### Example 4

The procedure of Example 1 was repeated 22 times. The average yield of recovered terephthalic acid was 89.3 mol %. All samples were homogenized and the resulting mixture analyzed for metal and nitrogen content. The resultant terephthalic acid contained 7.1 ppm of iron. The content of nitrogen is below 0.1 ppm and the color parameters are L* = 82.68, a* = - 1.46 and b* = 4.96.

### Example 5

The procedure of Comparative Experiment 2 was repeated except that 27 g of PET as in Comparative Experiment 1 and 150 g of water containing 3.6 wt % of ethylene glycol was used. The recovered terephthalic acid contained 46.4 ppm of iron.

### Example 6

The procedure of Example 5 was repeated except that 0.20 g of PVC (740 ppm) and 1.25 g of polyamide MXD6 was used. The resultant terephthalic acid contained 9.7 ppm of iron.

## Claims

1. A method of recovering aromatic dicarboxylic acids from aromatic polyester resins containing less than 2 500 ppm of halogen containing compounds, comprising the step of depolymerizing the aromatic polyester resin with water at temperatures in the range of 230 °C to 300 °C wherein the depolymerization reaction occurs in the presence of a polyamide wherein the polyamide is selected from the group consisting of the repeating unit of amino caproic acid and the reaction product of A and D, wherein A is a residue of a dicarboxylic acid comprising adipic acid, isophthalic acid, terephthalic acid, 1,4-cyclohexanedicarboxylic acid, or naphthalene dicarboxylic acid, and D is a residue of a diamine comprising m-xylylene diamine, p-xylylene diamine, hexamethylene diamine, ethylene diamine, or 1,4 cyclohexanedimethylamine.

2. A method according to claim 1, wherein the polyamide is present in the range of 0.1 to 10 weight percent.

3. A method according to claims 1 or 2, in which the depolymerization proceeds in the presence of 1 to 8 wt % of activated charcoal calculated to the amount of polyester resin.

4. A method according to claims 1 or 2, in which the reaction mixture is passed through a fixed-bed adsorber filled with activated charcoal at a temperature in the range of 50 °C below the depolymerization temperature of the polyester with water and the depolymerization temperature of the polyester with water.

5. A method according to claims 1 or 2, in which the ratio of the amount of water at the end of the depolymerization to the amount of aromatic polyester resin present in the reaction mixture is between 4 : 1 to 10 : 1.

6. A method according to claim 4, in which the water at the beginning of the reaction contains ethylene glycol at an amount less than or equal to 10 weight percent with respect to the water plus ethylene glycol mixture.

7. A method according to claim 5, wherein the aromatic dicarboxylic acid is separated from the reaction mixture cooled to the temperature in the range from 180 to 90 °C.

## Patentansprüche

1. Verfahren zur Rückgewinnung von aromatischen Dicarbonsäuren aus aromatischen Polyesterharzen, die weniger als 2.500 ppm halogenhaltiger Verbindungen enthalten, umfassend einen Schritt zur Depolymerisierung des aromatischen Polyesterharzes mit Wasser bei einer Temperatur im Bereich von 230 bis 300°C, worin die Depolymerisationsreaktion in Gegenwart eines Polyamids abläuft, wobei das Polyamid ausgewählt ist aus der Gruppe bestehend aus der Wiederholungseinheit von Aminocapronsäure und dem Reaktionsprodukt von A und D, worin A ein Rest einer Dicarbonsäure, umfassend Adipinsäure, Isophthalsäure, Terephthalsäure, 1,4-Cyclohexandicarbonsäure oder Naphthalindicarbonsäure, ist und D ein Rest eines Diamins, umfassend m-Xylylendiamin, p-Xylylendiamin, Hexamethylendiamin, Ethylendiamin oder 1,4-Cyclohexandimethylamin, ist.

2. Verfahren gemäss Anspruch 1, worin das Polyamid im Bereich von 0,1 bis 10 Gew.% vorliegt.

3. Verfahren gemäss Anspruch 1 oder 2, worin die Depolymerisation in Gegenwart von 1 bis 8 Gew.% Aktivkohle, bezogen auf die Menge von Polyesterharz, fortschreitet.

4. Verfahren gemäss Anspruch 1 oder 2, worin die Reaktionsmischung bei einer Temperatur im Bereich von 50°C unterhalb der Depolymerisationstemperatur des Polyesterharzes mit Wasser und der Depolymerisationstemperatur des Polyesterharzes mit Wasser durch einen Festbettadsorber geführt wird, der mit Aktivkohle gefüllt ist.

5. Verfahren gemäss Anspruch 1 oder 2, worin das Verhältnis der Menge an Wasser bei Ende der Depolymerisation zur Menge an aromatischem Polyesterharz, das in der Reaktionsmischung vorliegt, zwischen 4:1 bis 10:1 liegt.

6. Verfahren gemäss Anspruch 4, worin das Wasser zu Beginn der Reaktion Ethylenglykol in einer Menge von weniger als oder gleich 10 Gew.%, bezogen auf die Mischung von Wasser plus Ethylenglykol, enthält.

7. Verfahren gemäss Anspruch 5, worin die aromatische Dicarbonsäure von der Reaktionsmischung, gekühlt auf eine Temperatur im Bereich von 180 bis 90°C, abgetrennt wird.

## Revendications

1. Procédé de récupération d'acides dicarboxyliques aromatiques à partir de résines polyesters aromatiques contenant moins de 2500 ppm de composés halogénés, lequel procédé comporte une étape de dépolymérisation de la résine polyester aromatique, réalisée avec de l'eau et à des températures situées dans la gamme allant de 230 °C à 300 °C, dans lequel procédé la réaction de dépolymérisation a lieu en présence d'un polyamide, lequel polyamide est choisi dans l'ensemble des polyamides constitués de motifs répétés d'acide amino-caproïque ou de motifs répétés symbolisés par A-D, où A représente le résidu d'un acide dicarboxylique comprenant de l'acide adipique, de l'acide isophtalique, de l'acide téréphtalique, de l'acide cyclohexane-1,4-dicarboxylique ou un acide naphtalène-dicarboxylique et D représente le résidu d'une diamine comprenant de la méta-xylylène-diamine, de la para-xylylène-diamine, de l'hexaméthylène-diamine, de l'éthylène-diamine ou de la cyclohexane-1,4-bis(méthylamine).

2. Procédé conforme à la revendication 1, dans lequel le polyamide est présent en une proportion de 0,1 à 10 % en poids.

3. Procédé conforme à la revendication 1 ou 2, dans lequel la dépolymérisation s'effectue en présence de charbon actif, présent en une quantité représentant 1 à 8 % du poids de la résine polyester.

4. Procédé conforme à la revendication 1 ou 2, dans lequel on fait passer le mélange réactionnel dans un adsorbeur à lit fixe garni de charbon actif, à une température située dans la gamme allant de 50 °C au-dessous de la température de dépolymérisation du polyester par l'eau à cette température de dépolymérisation du polyester par l'eau.

5. Procédé conforme à la revendication 1 ou 2, dans lequel le rapport de la quantité d'eau à la fin de la dépolymérisation à la quantité de résine polyester aromatique présente dans le mélange réactionnel vaut de 4/1 à 10/1.

6. Procédé conforme à la revendication 4, dans lequel l'eau, au début de la réaction, contient de l'éthylèneglycol en une quantité inférieure où égale à 10 % du poids total du mélange d'eau et d'éthylèneglycol.

7. Procédé conforme à la revendication 5, dans lequel l'acide dicarboxylique aromatique est séparé du mélange réactionnel refroidi à une température située dans la gamme allant de 180 °C à 90 °C.
